(19) Europäisches Patentamt
European Patent Office
Office européen des brevets

(11) Veröffentlichungsnummer : **0 093 892**
**B1**

(12) **EUROPÄISCHE PATENTSCHRIFT**

(45) Veröffentlichungstag der Patentschrift : **28.05.86**

(21) Anmeldenummer : **83103657.9**

(22) Anmeldetag : **15.04.83**

(51) Int. Cl.⁴ : **C 07 C 85/02**, C 07 C 87/50, C 07 C 87/52, C 07 C 87/56, C 07 C 87/66, B 01 J 23/44, C 07 C 85/11// C07C119/12

(54) **Verfahren zur Herstellung von aromatischen Aminen.**

(30) Priorität : **22.04.82 DE 3215060**
**03.11.82 DE 3240548**

(43) Veröffentlichungstag der Anmeldung : **16.11.83 Patentblatt 83/46**

(45) Bekanntmachung des Hinweises auf die Patenterteilung : **28.05.86 Patentblatt 86/22**

(84) Benannte Vertragsstaaten : **CH DE FR GB LI**

(56) Entgegenhaltungen :
**EP-A- 0 050 229**
**EP-A- 0 051 805**
**EP-A- 0 051 806**
**US-A- 3 442 950**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Berthold, Rüdiger, Dr.**
**Geierfeld 68**
**D-6232 Bad Soden am Taunus (DE)**
Erfinder : **Müller, Werner Heinrich, Dr.**
**Taunusblick 5**
**D-6239 Eppstein/Taunus (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Aus J. pharm. Soc. Japan 80, 83 (1960) ist es bekannt, aromatische Amine aus cycloaliphatischen Ketonen und Ammoniak durch Umsetzung in der Gasphase an Cadmiumphosphat-sauren Ton-Katalysatoren herzustellen. Hierbei werden jedoch die aromatischen Amine in relativ geringen Ausbeuten erhalten, wobei mehr oder weniger — zum Teil überwiegend — die entsprechenden 2-Methyl-Pyridinbasen gebildet werden.

Die Erfindung betrifft ein Verfahren zur Herstellung von aromatischen Aminen durch Erhitzen von cycloaliphatischen Ketonen mit Ammoniak an Katalysatoren, das dadurch gekennzeichnet ist, daß man die Reaktionsteilnehmer in flüssiger Phase in einem ethergruppenhaltigen Lösungsmittel mit einem ein Edelmetall der 8. Nebengruppe des Periodensystems enthaltenden Katalysator auf Temperaturen von 150 bis 350 °C erhitzt.

Das erfindungsgemäße Verfahren liefert die aromatischen Amine in hohen Ausbeuten ; die entsprechenden 2-Methyl-Pyridinbasen werden lediglich in untergeordneten Mengen gebildet.

Bei der erfindungsgemäßen Umsetzung wird in situ das entsprechende Imin gebildet. Es ist deshalb auch möglich, für das erfindungsgemäße Verfahren direkt das Imin einzusetzen.

Aus dem Imin wird das aromatische Amin in einer Dehydrierungsreaktion gebildet, wobei beim Einsatz eines gesättigten cycloaliphatischen Imins zwei Mol, beim Einsatz eines einfach ungesättigten cycloaliphatischen Imins ein Mol Wasserstoff abgespalten werden. In einer bevorzugten Ausgestaltung der Erfindung wird deshalb dem Reaktionsgemisch ein Oxidationsmittel zugesetzt, das den abgespaltenen Wasserstoff zu Wasser oxidiert. Hierdurch wird vermieden, daß in einer Nebenreaktion das eingesetzte Keton zum Alkohol reduziert wird.

Besonders geeignete Oxidationsmittel sind aromatische Nitroverbindungen. Zweckmäßigerweise wird die aromatische Nitroverbindung so ausgewählt, daß das entstehende aromatische Amin leicht vom Verfahrensprodukt abzutrennen ist, beispielsweise destillativ aufgrund der unterschiedlichen Siedepunkte. Besonders vorteilhaft ist es, wenn als aromatische Nitroverbindung diejenige eingesetzt wird, die bei der Reduktion das Verfahrensprodukt selbst liefert. Dies setzt in der Praxis voraus, daß diese entsprechende Nitroverbindung ihrerseits leicht zugänglich ist. Wenn dies nicht der Fall ist, wählt man im allgemeinen zweckmäßigerweise das Nitrobenzol, da hierbei das relativ leicht flüchtige Anilin gebildet wird.

Vorteilhaft setzt man nur so viel aromatische Nitroverbindung ein, wie im Laufe der Umsetzung verbraucht wird, um die Isolierung des Verfahrensprodukts nicht zu erschweren. Ein eventueller Überschuß kann aber in bekannter Weise, beispielsweise durch Ausrühren der Reaktionslösung mit Zinkstaub, beseitigt werden.

Als Ausgangsmaterialien kommen sowohl gesättigte als auch ungestättigte ein- oder mehrkernige Ketone bzw. Imine in Betracht, die inerte Substituenten tragen können. Selbstverständlich müssen diese Substituenten so angeordnet sein, daß eine Aromatisierung des Moleküls möglich ist. Ausgeschlossen sind auch « sperrige » Substituenten in den zur Keto- bzw. Imingruppe benachbarten Stellungen, da hierdurch der Kontakt zwischen dem Keton- bzw. Iminmolekül und dem Katalysator be- bzw. verhindert würde.

Geeignete Ausgangsmaterialien sind Cyclohex-2-en-1-on(imin)e und Cyclohexanon(imin)e bzw. die entsprechenden Tetrahydronaphthalin-1-on(imin)e, da diese sehr selektiv zu den entsprechenden primären aromatischen Aminen führen. Bevorzugt sind Cyclohex-2-en-1-on(imin)e bzw. Cyclohexanon(imin)e der Formeln

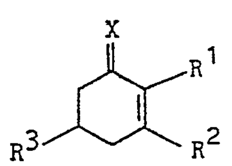 (I) bzw. 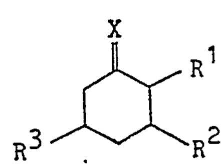 (II)

worin X O oder NH bedeutet, und

worin $R^1$, $R^2$ und $R^3$ gleich oder verschieden sind und jeweils Wasserstoff, niederes Alkyl oder Phenyl bedeuten. Besonders bevorzugt sind Verbindungen der Formeln I bzw. II, worin $R^1$H und $R^2$ und $R^3$H oder Alkyl mit 1-4 C-Atomen, insbesondere Methyl, bedeuten.

Die Ketone sind leicht aus den entsprechenden Acylessigestern und Aldehyden zugänglich, beispielsweise nach dem in der DE-OS 26 54 850 beschriebenen Verfahren.

Da es sich bei dem erfindungsgemäßen Verfahren um eine Reaktion im heterogenen System handelt, ist es zweckmäßig, ständig für eine gute Durchmischung zu sorgen. Diese wird durch entsprechendes Rühren erreicht.

Bevorzugte Reaktionstemperaturen liegen bei 180 bis 260 °C, da in diesem Bereich die Reaktion rasch und im allgemeinen mit hoher Selektivität abläuft. Je nach Art des eingesetzten Lösungsmittels wird bei Drücken von 5 bis 200, insbesondere von 70 bis 130, bar gearbeitet, wobei Druck und Temperatur

zweckmäßig so aufeinander abgestimmt sind, daß genügend flüssige Phase vorhanden ist und die erwünschte Reaktionstemperatur aufrechterhalten wird.

Als Lösungsmittel eignen sich grundsätzlich alle Ether. Bevorzugt sind aliphatische Ether, also solche Ether, die mindestens eine aliphatische Gruppe enthalten. Vorzugsweise werden Ether mit einem Siedepunkt von mindestens etwa 80 °C eingesetzt, da bei niedriger siedenden Ethern relativ hohe Drücke erforderlich sind, um eine genügend hohe Reaktionstemperatur zu erreichen, bei der die Reaktion in annehmbarer Zeit zu Ende geführt werden kann.

Geeignet sind höher siedende Dialkylether, beispielsweise Di-n-butylether, Alkyl-aryl-ether wie Anisol und Phenetol, insbesondere jedoch Polyether, die sich vom Ethylen- und/oder Propylenglykol ableiten. Als solche Polyether kommen im einfachsten Fall die niederen Dialkylether des Ethylenglykols und Propylenglykols in Betracht, vorzugsweise jedoch niedere Dialkylether von Polyglykolen wie Ethylendi-, -tri- oder -tetraglykol oder von höheren Polyglykolen, weiterhin auch Monoarylether, z. B. Anlagerungsprodukte von Ethylenoxid an Alkylphenole wie Nonylphenol oder Tributylphenol, deren freie Hydroxygruppe mit niederen Alkanolen verethert sein kann. Als Ether kommen jedoch auch höhere Polyglykole mit freien Hydroxy-Endgruppen in Betracht, bevorzugt sind jedoch ihre niederen Dialkylether mit bis zu 6 Kohlenstoffatomen in den Ethergruppen, insbesondere die Methyl- und Ethylether.

Bevorzugt wird ein Ether eingesetzt, der tiefer als das entstehende Amin siedet. So kann man das Lösungsmittel laufend abdestillieren und — um Lösungsmittel einzusparen — in den Prozeß zurückführen, wobei das Amin aus dem Sumpf ausgeschleust wird. Überschüssiges Ammoniak wird zurückgewonnen. Diese Verfahren lassen sich auch vollkontinuierlich ausgestalten. Hierbei wird beispielsweise eine Lösung des Gemisches aus Keton und Ammoniak in demselben Lösungsmittel, das auch zur Suspendierung des Katalysators verwendet wird, kontinuierlich über einen Vorheizer in den Reaktor eingebracht, während gleichzeitig eine entsprechende Menge der Reaktionsmischung, die das gebildete Amin enthält, ausgetragen wird. Der Katalysator wird hierbei beispielsweise mittels einer Fritte im Reaktor zurückbehalten oder nach Abtrennung, z. B. mittels eines Dekanters, in den Reaktor zurückgeführt. Das Lösungsmittel wird nach destillativer Abtrennung vom gebildeten Amin ebenfalls zurückgeführt.

Die Katalysatoren sind entweder Skelett- oder Trägerkatalysatoren, die als wirksames Metall zweckmäßig Ruthenium, Rhodium, Iridium, insbesondere jedoch Palladium oder Platin oder mehrere dieser Metalle enthalten. Als Träger für die Katalysatoren kommen üblicherweise Kohle, Siliziumdioxide, Aluminiumoxid, Aluminiumsilikate, Mischoxide wie Chromoxid-Aluminiumoxid oder Spinelle, Bariumsulfat oder mineralische Träger wie Zeolithe in Betracht. Die Konzentration des Edelmetalls auf dem Träger beträgt 0,05 bis 10 Gew.-%, vorzugsweise 0,2 bis 5 Gew.-%, insbesondere 0,5 bis 2,5 Gew.-%.

Wenn die handelsüblichen Trägerkatalysatoren eingesetzt werden, wählt man eine Katalysator-Teilchengröße von 0,01 bis 5 mm, vorzugsweise 0,05 bis 1 mm. Abhängig vom Lösungsmittel und vom Katalysator kann die Reaktionssuspension 0,1 bis 40 % Trägerkatalysator, bezogen auf das Gewicht des flüssigen Reaktionsmediums, enthalten. Bevorzugt sind 1 bis 30 %, bezogen auf das Gewicht des Lösungsmittels.

Die Aktivität des Katalysators nimmt in Laufe der Reaktion allmählich ab, wobei im gleichen Maße Nebenprodukte gebildet werden. Diese Nebenprodukte bleiben bei der destillativen Abtrennung des Amins im Rückstand, z. T. werden sie auch mit dem Lösungsmittel als Vorlauf abgetrennt. Unter den optimalen Bedingungen der Xylidinherstellung entstehen nicht mehr als 1 % d. Th. dieser Substanzen.

In den folgenden Beispielen beziehen sich die Prozentangaben auf das Gewicht, sofern nichts anderes angegeben ist.

## Beispiel 1

In einem 1 l-Edelstahl-Hubrührautoklav wurden 250 g Ethylenglykol-Dimethylether, 250 g 3,5-Dimethyl-Cyclohex-2-en-1-on und 82 g Nitrobenzol vorgelegt. Dazu gab man 5 g eines Katalysators, der aus 5 % Palladium auf Aluminiumoxid-Pulver besteht. Nach dem Verschließen des Autoklaven drückte man noch 200 g flüssiges Ammoniak auf. Nun wurde auf 200 °C erhitzt, wobei sich ein Druck von 90 bis 100 bar aufbaute. Bei dieser Temperatur wurde eine Stunde gerührt. Nach dem Abkühlen auf Raumtemperatur und Ablassen des überschüssigen Ammoniakdruckes wurde die braune Reaktionslösung durch Absaugen vom Katalysator befreit. Der Katalysator wurde mit Ethylenglykol-Dimethylether gewaschen. Er kann in einer folgenden Operation wieder eingesetzt werden. Nun destillierte man den Ethylenglykol-Dimethylether ab. Dies kann ebenfalls bei leicht reduziertem Druck erfolgen. Der Rückstand hatte nach gaschromatographischer Untersuchung folgende Zusammensetzung :

| | |
|---|---|
| sym-Kollidin | 0,82 % |
| Unbekannte | 0,21 % |
| Anilin | 21,46 % |
| Nitrobenzol | — |
| Dimethylcyclohexenon | — |
| sym. m-Xylidin | 75,2 % |
| Summe späteluierte | 2,3 % |

Dieses Gemisch ließ sich durch fraktionierte Vakuumdestillation trennen. Man erhielt :

| | |
|---|---|
| Vorlauf(sym-Kollidin, wenig Anilin und Unbekannte) Kp bei 27 mbar bis 83 °C | 5,8 g |
| Anilin Kp bei 27 mbar 83-87 °C | 65 g |
| Xylidin Kp bei 33 mbar 110-115 °C | 222 g (= 1,84 Mol = 92 % d. Th.) |
| Rückstand | 16,3 g |

Beispiel 2

In einem 1 l Hubrührautoklav wurden 64 g Ethylenglykol-Dimethylether, 250 g 3,5-Dimethylcyclohex-2-en-1-on, 84 g Nitrobenzol und 10 g eines Katalysators vorgelegt, der aus 5 % Palladium auf Aluminiumoxid-Pulver besteht. Nach dem Verschließen des Autoklaven drückte man 250 g Ammoniak auf. Nun wurde auf 225 °C erhitzt, wobei sich ein Druck von etwa 125 bar aufbaute. Bei dieser Temperatur wurde eine Stunde gerührt. Die Aufarbeitung erfolgte wie im Beispiel 1 angegeben. Man erhielt 306 g eines Gemisches folgender Zusammensetzung :

| | |
|---|---|
| sym-Kollidin | 0,75 % = 2,3 g |
| Anilin | 25,5 % = 78,0 g |
| sym. m-Xylidin | 68,5 % = 209,6 g |
| Unbekannte Späteluierte | 5,3 % = 16,2 g |

Dieses Gemisch ließ sich durch Vakuumdestillation trennen :

| | |
|---|---|
| Vorlauf Kp bei 27 mbar bis 83 °C | 9,2 g (Kollidin, Anilin) |
| Anilin Kp bei 27 mbar 83-87 °C | 70 g |
| sym.-m-Xylidin Kp bei 27 mbar 110-115 °C | 250 g (= 1,69 Mol = 84,7 % d. Th.) |
| Rückstand | 20 g |

Beispiel 3

(Vergleichsbeispiel ohne ethergruppenhaltiges Lösungsmittel) :

250 g 3,5-Dimethylcyclohex-2-en-1-on, 82 g Nitrobenzol,
5 g Palladiumkontakt (5 % Pd auf $Al_2O_3$) und
200 g Ammoniak wurden wie im Beispiel 1 angegeben eine Stunde bei 225 °C gerührt und dann entsprechend aufgearbeitet. Man erhielt 322 g eines Gemisches, welches nur 17,9 % sym. m-Xylidin enthielt, entsprechend 56,6 g (= 0,48 Mol = 24 % d. Th.).

Beispiele 4 bis 11

250 g Glykoldimethylether, 0,2 Mol eines Cyclohexanon- bzw. Cyclohexenonderivates, 10 g Nitrobenzol, 5 bis 10 g eines Palladiumkontaktes, der 5 % Pd auf Kohle bzw. $Al_2O_3$ enthält, und 100 g wasserfreier Ammoniak wurden wie im Beispiel 1 beschrieben, eine Stunde bei 200 °C gut gerührt und wie in Beispiel 1 beschrieben aufgearbeitet. Die Ergebnisse sind in der nachfolgenden Tabelle zusammengefaßt.

(Siehe Tabelle Seite 5 f.)

| Bsp. | Einsatzprodukt | Ausbeute an Reaktions-gemisch | Reaktionsprodukt Konstitution | Gehalt an Amin [g] | [%] | Ausbeute [% d. Th.] |
|---|---|---|---|---|---|---|
| 4 | 5-Methylcyclohex-2-en-1-on | 324 g | m-Toluidin | 19,8 | 6,1 | 92,5 % |
| 5 | 5-Propyl-3-methyl-cyclohex-2-en-1-on | 270 g | 3-Propyl-5-methyl-anilin | 21 | 7,7 | 70,0 % |
| 6 | 5-Phenyl-3-methyl-cyclohex-2-en-1-on | 325 g | 3-Phenyl-5-methyl-anilin | 22 | 6,75 | 60 % |
| 7 | Cyclohexanon | 329 g | Anilin | 27,6 | 8,4 | 89 % |
| 8 | 2-Methylcyclohexanon | 327 g | o-Toluidin | 16,1 | 4,9 | 75 % |
| 9 | 3-Methylcyclohexanon | 333 g | m-Toluidin | 15,4 | 4,6 | 72 % |
| 10 | 4-Methylcyclohexanon | 310 g | p-Toluidin | 10,7 | 3,45 | 50 % |
| 11 | -Tetralon | 327 g | α-Naphthylamin | 21,8 | 6,7 | 73 % |

**Patentansprüche**

1. Verfahren zur Herstellung von aromatischen Aminen durch Erhitzen von cycloaliphatischen Ketonen mit Ammoniak an einem Katalysator, dadurch gekennzeichnet, daß man die Reaktionsteilnehmer in flüssiger Phase in einem ethergruppenhaltigen Lösungsmittel mit einem ein Edelmetall der 8. Nebengruppe des Periodensystems enthaltenden Katalysator auf Temperaturen von 150 bis 350 °C erhitzt.

2. Verfahren nach Anspruch 1, dadurch gekennzeichnet, daß man anstelle des cycloaliphatischen Ketons und Ammoniak das entsprechende Imin einsetzt.

3. Verfahren nach Anspruch 1 und 2, dadurch gekennzeichnet, daß man dem Reaktionsgemisch ein Oxidationsmittel zusetzt.

4. Verfahren nach Anspruch 3, dadurch gekennzeichnet, daß das Oxidationsmittel eine aromatische Nitroverbindung ist.

5. Verfahren nach Anspruch 4, dadurch gekennzeichnet, daß diejenige Nitroverbindung eingesetzt wird, die bei der Reduktion zum Verfahrensprodukt führt.

6. Verfahren nach Anspruch 4, dadurch gekennzeichnet, daß eine Nitroverbindung eingesetzt wird, die bei der Reduktion zu einem aromatischen Amin führt, das leicht vom Verfahrensprodukt abgetrennt werden kann.

7. Verfahren nach Anspruch 1 und 3 bis 6, dadurch gekennzeichnet, daß man als cycloaliphatisches Keton ein Cyclohexanon, Cyclohex-2-en-1-on bzw. das entsprechende Tetrahydronaphthalin-1-on einsetzt, vorzugsweise eine Verbindung der allgemeinen Formeln

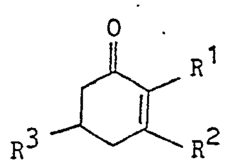 (Ia) bzw. 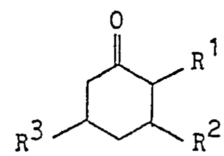 (IIa)

worin $R^1$, $R^2$ und $R^3$ gleich oder verschieden sind und jeweils Wasserstoff, niederes Alkyl oder Phenyl bedeuten.

8. Verfahren nach Anspruch 2 bis 6, dadurch gekennzeichnet, daß man als cycloaliphatisches Imin ein Cyclohexanimin, Cyclohex-2-en-1-imin bzw. das entsprechende Tetrahydronaphthalin-1-imin einsetzt, vorzugsweise eine Verbindung der allgemeinen Formeln

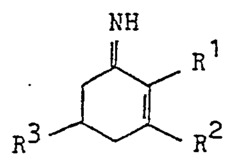 (Ib) bzw. 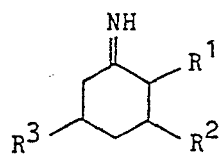 (IIb)

worin $R^1$, $R^2$ und $R^3$ gleich oder verschieden sind und jeweils Wasserstoff, niederes Alkyl oder Phenyl bedeuten.

9. Verfahren nach Anspruch 7 und 8, dadurch gekennzeichnet, daß in den Formeln Ia, Ib, IIa und IIb $R^1$H und $R^2$ und $R^3$H oder Alkyl mit 1-4 C-Atomen, insbesondere Methyl, bedeuten.

**Claims**

1. A process for preparing aromatic amines by heating cycloaliphatic ketones together with ammonia using a catalyst, which comprises heating the reactants in the liquid phase in an ether group containing solvent together with a catalyst containing a noble metal of secondary group VIII of the periodic table of the elements to temperatures of 150 to 350 °C.

2. The process as claimed in claim 1, wherein the corresponding imine is used in place of the cycloaliphatic ketone and ammonia.

3. The process as claimed in claims 1 and 2, wherein an oxidizing agent is added to the reaction mixture.

4. The process as claimed in claim 3, wherein the oxidizing agent is an aromatic nitro compound.

5. The process as claimed in claim 4, wherein that nitro compound is used which on reduction gives the product.

6. The process as claimed in claim 4, wherein the nitro compound used leads on reduction to an aromatic amine which is easily separated from the product.

7. The process as claimed in claims 1 and 3 to 6, wherein the cycloaliphatic ketone used is a cyclohexanone, cyclohex-2-en-1-one or the corresponding tetrahydronaphthalene-1-one, preferably a compound of the general formulae

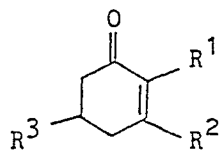 (Ia) or 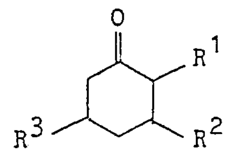 (IIa)

in which $R^1$, $R^2$ and $R^3$ are identical or different and each denotes hydrogen, lower alkyl or phenyl.

8. The process as claimed in claims 2 to 6, wherein the cycloaliphatic imine used is a cyclohexanimine, cyclohex-2-en-1-imine or the corresponding tetrahydronaphthalene-1-imine, preferably a compound of the general formulae

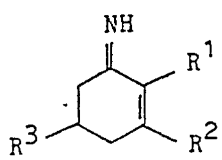 (Ib) or 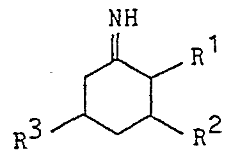 (IIb)

in which $R^1$, $R^2$ and $R^3$ are identical or different and each denotes hydrogen, lower alkyl or phenyl.

9. The process as claimed in claims 7 and 8, wherein, in the formulae Ia, Ib, IIa and IIb, $R^1$ denotes H and $R^2$ and $R^3$ denote H or alkyl having 1-4 carbon atoms, in particular methyl.

**Revendications**

1. Procédé de préparation d'amines aromatiques par chauffage de cétones cycloaliphatiques avec de l'ammoniac sur un catalyseur, procédé caractérisé en ce que l'on chauffe les réactifs en phase liquide dans un solvant à groupes éthers avec un catalyseur comprenant un métal noble du sous-groupe 8 de la classification périodique des éléments, à des températures de 150 à 350 °C.

2. Procédé selon la revendication 1 caractérisé en ce que, à la place de la cétone cycloaliphatique et d'ammoniac, on part de l'imine correspondante.

3. Procédé selon la revendication 1 ou 2 caractérisé en ce que l'on ajoute un agent oxydant au mélange réactionnel.

4. Procédé selon la revendication 3 caractérisé en ce que l'oxydant est un composé nitré aromatique.

5. Procédé selon la revendication 4 caractérisé en ce que l'on utilise un composé nitré dont la réduction donne le produit du procédé.

6. Procédé selon la revendication 4 caractérisé en ce que l'on utilise un composé nitré dont la réduction donne une amine aromatique pouvant être facilement séparée du produit.

7. Procédé selon l'une quelconque des revendications 1 et 3 à 6, caractérisé en ce que l'on part, comme cétone cycloaliphatique, d'une cyclohexanone, d'une cyclohex-2-ène-1-one ou de la tétrahydronaphtalène-1-one correspondante, de préférence d'un composé de formule générale

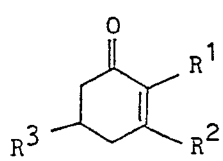 (Ia) ou 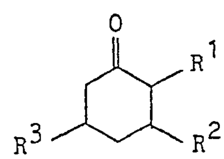 (IIa)

dans lesquelles $R^1$, $R^2$ et $R^3$, qui peuvent être identiques ou différents les uns des autres, représentent chacun l'hydrogène, un alkyle inférieur ou le groupe phényle.

8. Procédé selon l'une quelconque des revendications 2 à 6, caractérisé en ce que l'on part, comme imine cycloaliphatique, d'une cyclohexane-imine, d'un cyclohex-2-ène-1-imine ou de la tétrahydronaphtalène-1-imine correspondante, de préférence d'un composé de formule générale

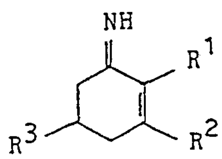 (Ib) ou 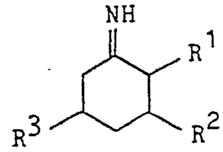 (IIb)

dans lesquelles $R^1$, $R^2$ et $R^3$, qui peuvent être identiques ou différents, représentent l'hydrogène, un alkyle inférieur ou le groupe phényle.

9. Procédé selon la revendication 7 ou 8, caractérisé en ce que dans les formules Ia, Ib, IIa et IIb, $R^1$ représente l'hydrogène et $R^2$ et $R^3$ représentent chacun l'hydrogène ou un alkyle en $C_1$ à $C_4$, en particulier le groupe méthyle.